# EUROPEAN PATENT SPECIFICATION

(11) **EP 0 325 199 B1**
(45) Date of publication and mention of the grant of the patent: **27.10.1993**
(21) Application number: 89100714.8
(22) Date of filing: 17.01.1989
(51) Int. Cl.: A61K 31/335

(54) **Fumagillin as angiostatic agent**
Fumagillin als angiostatisches Mittel
Fumagilline comme agent angiostatique

(30) Priority: 19.01.1988 US 145407; 25.03.1988 US 173305
(43) Date of publication of application: 26.07.1989
(73) Proprietor: Takeda Chemical Industries, Ltd., Chuo-ku, Osaka (JP); CHILDREN'S MEDICAL CENTER CORPORATION, Boston Massachusetts 02115 (US)
(72) Inventor: Folkman, Judah, Brookline Massachusetts 02146 (US); Ingber, Donald, Boston Massachusetts 02116 (US); Fujita, Takeshi, Takarazuka Hyogo 665 (JP); Kanamaru, Tsuneo, Takatsuki Osaka 569 (JP)
(74) Representative: von Kreisler, Alek, Dipl.-Chem.

(56) References cited:
- CHEMICAL ABSTRACTS, vol. 61, no.2, 20 July 1964, Columbus, OH (US);M.N. SEMENOV et al., no. 1709h#
- CHEMICAL ABSTRACTS, vol. 63, no. 2, 19 July 1965, Columbus, OH (US); N.B. TURSUNKHODZHAEV, no. 2251e#
- CHEMICAL ABSTRACTS, vol. 70, no. 9, 03 March 1969, Columbus, OH (US); M.M. MAEVSKII et al., no. 36365g#
- SCIENCE, vol. 221, no. 4612, 19 August 1983; J. FOLKMAN et al., pp. 719-725#
- SCIENCE, vol. 230, no. 4732, 20 December 1985; R. CRUM et al., pp. 1375-1378#
- J. CELL. BIOL., vol. 107, no. 6, part 3, December 1988; J. FOLKMAN et al., p. 579a, no. 3277#

## Description

The present invention relates to the use of fumagillin for the preparation of medicaments for the prophylaxis or treatment of angiogenesis-related diseases induced by abnormally stimulated neovascularization in mammals. The invention also relates to certain pharmaceutical compositions comprising fumagillin and potentiators of angiogenesis inhibition.

### Background of the Invention

Angiogenesis is a process by which new capillary blood vessels are formed. This process occurs normally, e.g., during ovulation and formation of the placenta. It also occurs pathologically in wound healing, as well as in a variety of diseases where uncontrolled or rampant capillary growth is the cause of extensive tissue damage. Examples of the latter are in ophthalmology: diabetic retinopathy, retrolental fibroplasia, corneal graft neovascularization, neovascular glaucoma, ocular tumors and trachoma, where neovascularization may lead to blindness; in dermatology: psoriasis and pyogenic granuloma; in pediatrics: hemangioma, angiofibroma, and hemophiliac joints; in surgery: hypertrophic scars, wound granulation and vascular adhesions; in internal medicine: rheumatoid arthritis, where new vessels in the joint may destroy articular cartilage and scleroderma; in cardiology: atherosclerotic plaque; and in cancer: many kinds of carcinomas and sarcomas, where progressive growth is dependent upon the continuous induction of angiogenesis by these tumor cells.

The realization that tumors, as well as many non-neoplastic diseases, are angiogenesis-dependent has led to a search for angiogenesis inhibitors that might be used therapeutically (See e.g., J. Folkman; Advances in Cancer Research Vol. 43, pp 175-203, 1985 ed. George Klein and Sidney Weinhouse).

Chemical Abstracts Volume 61, No. 2, July 20, 1964, Abstract No. 1709h discloses the isolation of fumagillin and its use in the treatment of nosematosis of bees. The potassium salt of fumagillin was reported to have an inhibitory effect on carcinoma and sarcoma of rats and mice.

Chemical Abstracts Volume 63, No. 2, July 19, 1965, Abstract No. 2251e, discloses that fumagillin acted directly on tumor cells by reducing the amount of RNA.

Chemical Abstracts Volume 70, No. 9, March 3, 1969, page 206, Abstract No. 36365g, discloses fumagillin as an anti-tumor antibiotic.

However, none of the above Chemical Abstract references disclose or suggest the anti-angiogenesis activity of fumagillin.

Fumagillin is a known compound which has been used as an antimicrobial and antiprotozoal. Its physicochemical properties and method of production are well documented [Production; USP 2,803,586: Structure; Proc. Nat. Acad. Sci. USA, 48, 733-735 (1962)].

Fumagillin has a molecular formula of C₂₆H₃₄O₇. Its molecular weight is 458.53. Yellow needles from methanol extraction have an mp of about 194-195°C. Fumagillin has the following structure:

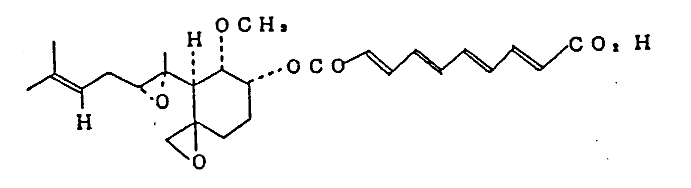

### Summary of the Invention

The present invention relates to the use of the fumagillin, obtainable from Aspergillus fumigatus, as an angiogenesis inhibitor. Fumagillin shows strong angiogenesis inhibitory activity and may be used to treat a variety of diseases induced by abnormally stimulated angiogenesis including diabetic retinopathy and trachoma. The present invention also relates to certain pharmaceutical compositions comprising fumagillin and agents which potentiate angiogenesis inhibition such as heparin and sulfated cyclodextrin, particularly beta cyclodextrin tetradecasulfate.

### Brief Description of the Drawings

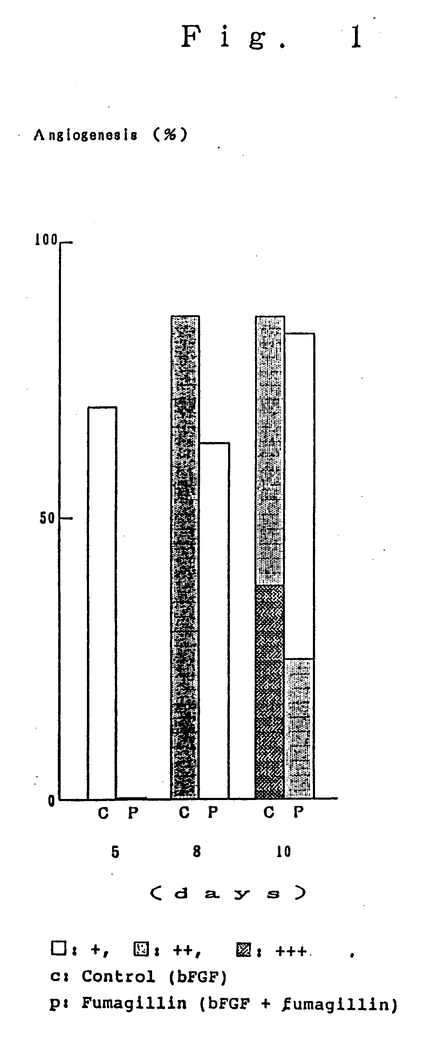

Fig. 1 illustrates the inhibition of neovascularization stimulated by bFGF by treatment with fumagillin in accordance with the rat corneal micropocket assay, which is disclosed in detail in Example 4.

### Detailed description of the invention

The present invention provides the use of fumagillin or a pharmaceutically acceptable salt thereof for preparing a medicament for inhibiting or preventing angiogenesis in mammals. The invention also provides certain pharmaceutical compositions comprising fumagillin or its salt, and an agent which potentiates angiogenesis inhibition such as heparin and sulfated cyclodextrins such as beta-cyclodextrin tetradecasulfate.

As the salt, inorganic salts such as alkali metal salt, e.g. sodium salt, potassium salt, alkaline-earth metal salt, e.g. calcium salt, and ammonium salt are preferred.

On the basis of its strong angiogenesis inhibitory activity, fumagillin and its salts are particularly useful for prophylaxis and treatment of diseases in the fields of ophthalmology, dermatology, pediatrics, surgery and cardiology.

Thus, fumagillin and its salts may be used for prophylaxis and/or treatment of neovascularization in diabetic retinopathy, retrolental fibroplasia, corneal graft neovascularization, neovascular glaucoma, ocular tumors, and trachoma; dermatological psoriasis and pyogenic granuloma; childrens hemangioma, angiofibroma and hemophiliac joints; and hypertrophic scars, wound granulation, vascular adhesions, rheumatoid arthritis, scleroderma and atherosclerotic plaque. Among these diseases, fumagillin and its salts are especially effective against diabetic retinopathy.

The use of fumagillin in accordance with the present invention has been found to be low in toxicity and is safely administered orally or parenterally to mammals (e.g. rat, rabbit, monkey, man) in forms of e.g. tablets, granules, capsules, injectable solutions, topical creams, and eye-drops.

To treat diabetic retinopathy, for example, fumagillin is administered orally in an amount of from about 1 mg/kg to 200 mg/kg per day, preferably in an amount of from about 2 mg/kg to 100 mg/kg per day. For oral administration, 5 mg to 100 mg of fumagillin or its salts may be formulated as a tablet or a capsule together with a pharmaceutically acceptable carrier, diluent or other suitable vehicle.

Fumagillin may also be administered subcutaneously or intravenously in an amount of from about 0.1 mg/kg to 20 mg/kg per day to an adult in the form of a pharmaceutical acceptable composition. Preferably, it is administered in an amount of from about 0.2 mg/kg to 10 mg/kg per day. The preferred form of fumagillin for intravenous administration is as a sodium salt.

Fumagillin may also be administered topically. For example, to treat eye-related angiogenesis diseases, fumagillin, especially as a salt, may be administered in the form of eye-drops. One to a few drops per dose is administered to the eye with a frequency of 1 to about 4 times a day according to the patient's condition. Preferably, the eye-drops are prepared by dissolving a fumagillin salt in distilled water to make a concentration of 0.001 to 3% (w/v). The solution also preferably contains an isotonizing agent, a preservative, or a thickening agent and is adjusted to a pH of from about 5 to 9.

In accordance with another aspect of the present invention, it has been found that certain agents potentiate the angiogenesis inhibitory activity of fumagillin. Such agents include heparin and a group of compounds known as sulfated cyclodextrins.

Heparin, an alpha, beta glycosidically linked highly sulfated copolymer of uronic acid and glycosamine, has been used clinically as an anticoagulant for half a century. Despite its importance and widespread use, both the exact structure of heparin and the precise nature by which it acts in blood anticoagulation have not been discovered. Much of the difficulty in determining the structure of heparin results from the fact that it is not a homogeneous well-defined substance. Heparin is polydisperse with a molecular weight range from 5,000 to 40,000. Within a given chain, there are structural variations such as the varying degrees of sulfation, N-acetylation and C-5 epimerization in the uronic acid residue.

Cyclodextrins are natural cyclic compounds consisting of six (alpha), seven (beta) or eight (gamma) D-glucose units. It has a donut-shaped molecular structure which provides a cavity whereby clathrates may form with guest molecules of suitable size. The interior of the cavity consists largely of uniformly spaced bridging acetal oxygen atoms. One end of the cavity is edged with -CH₂OH Groups (one per glucose unit) and the other rim is similarly edged with secondary -CHOH Groups. The cavity contains water molecules hydrogen bonded to the interior oxygen atoms.

The alpha, beta, and gamma cyclodextrin sulfate salts are all usable as potentiating agents of fumagillin in accordance with the present invention. Beta-cyclodextrin salts are such as beta-cyclodextrin tetradecasulfate are preferred.

As discussed in more detail below, the angiogenesis inhibitory activity of fumagillin is markedly potentiated by sulfated polysaccharides such as heparin and beta cyclodextrin tetradecasulfate.

Thus, such agents may be used in conjunction with fumagillin in the prophylaxis or treatment of angiogenesis-related diseases. As the skilled artisan will appreciate, the relative amount of such potentiating agents to fumagillin may vary depending on a number of factors, including the patient's condition and administration route. In general, the ratio of potentiating agent to fumagillin by weight is between about 1:10 to 30:1, preferably from about 1:3 to 10:1.

The invention will be further illustrated with reference to the following examples which will aid in the understanding of the present invention, but which are not to be construed as a limitation thereof.

### Example 1

### Shell-less Chorioallantoic Membrane Assay

The shell-less chorioallantoic membrane (CAM) assay was carried out by the method of Taylor and Folkman (S. Taylor and J. Folkman; Nature, 297, 307 (1982)) with a slight modification as follows: 3-days chick embryos were removed from their shells and cultured in plastic cups on hammocks of plastic wrap. The sodium salt of fumagillin 10 µg, along with acidic FGF 200 ng (bovine brain, R & D Systems, Inc.), and methylcellulose 30 µg (Fisher Scientific Co., 4000 centipoise) was placed on plastic disks (polypropylene, 6 mm in diameter). After the solution had dried, the disks were placed on the CAM of 10-day embryos. Three days later, inhibition of neovascular formation by fumagillin was observed under a stereoscope (X 20, SMZ-10, Nikon), and compared to the control disk containing acidic FGF 200 ng as a stimulant of angiogenesis and methylcellulose 30 µg without fumagillin. Fumagillin showed angiogenesis inhibitory activity by the CAM assay (Table 1).

**Table 1**

| Angiogenesis inhibitory activity of fumagillin by CAM assay. | |
|---|---|
| Number of disk showing angiogenesis inhibition/number of disk tested | |
| Exp. 1 | 3/3 |
| Exp. 2 | 2/6 |
| Exp. 3 | 6/9 |

### Example 2

### Shell-less Chorioallantoic Membrane Assay

The shell-less chorioallantoic membrane (CAM) assay was carried out by the method of Folkman et al. (R.Crum. S.Szabo and J.Folkman; Science. 230. 1375 (1985)) as follows: three-day chick embryos were removed from their shells to petri dishes (Falcon 1005) under sterile hood and cultured for further 3 days.

The sodium salt of fumagillin and/or sulfated polysaccharide (e.g. heparin, beta cyclodextrin tetradecasulfate) were dissolved to a 0.45% methylcellulose aqueous solution and aliquots of 10 µl were pipetted onto Teflon® rods. After the solution had dried, the methylcellulose disks (about 2 mm in diameter) containing test compounds, thus prepared, were implanted on the CAM of 6-day embryos.

After cultures for 48 - 72 hours, formations of avascular zones around the disks were observed with a stereoscope.

Percent of avascular zones was calculated by counting the disks forming avascular zones per the total disks tested. In each group there were 8 - 16 embryos.

As shown in Table 2, Fumagillin showed strong angiogenesis inhibitory activity by the CAM assay. Angiogenesis inhibitory activity of fumagillin was markedly potentiated by sulfated polysaccharides: heparin and beta cyclodextrin tetradecasulfate. Hydrocortisone does not potentiate and may suppress the effects of fumagillin.

**Table 2**

| Angiogenesis inhibitory activity of fumagillin and potentiation by sulfated polysaccharides. | | |
|---|---|---|
| compound tested / disk | | percent of avascular zones |
| fumagillin sodium salt | 50 µg | 57 % |
| | 40 µg | 72 % |
| | 30 µg | 62 % |
| | 20 µg | 66 % |
| | 10 µg | 75 % |
| fumagillin sodium salt + hydrocortisone | 10 µg | 40 % |
| | 60 µg | |
| fumagillin sodium salt + heparin | 10 µg | 100 % |
| | 50 µg | |
| fumagillin sodium salt | 5 µg | 40 % |
| fumagillin sodium salt + beta cyclodextrin tetradecasulfate | 5 µg | 70 % |
| | 25 µg | |

### Example 3

### Mouse dorsal air sac assay

The angiogenesis assay by mouse dorsal air sac method (MDA) was carried out by the method below which is a modification of the original rat dorsal air sac assay developed by Folkman et al. (Folkman, J. et al.; J.Exp.Med.,133, 275 (1971)). Specifically, Millipore® chambers, equipped with a Millipore® filter with a pore size of 0.45 µm (Millipore Corp.), were filled with 5 x 10⁶ sarcoma 180 cells in 0.15 ml of saline. The control contained the same volume of saline.

Under Nembutal anesthesia, each mouse received both the control and the tumor cell-containing chambers in their dorsal air sac produced by the method mentioned above. Fumagillin was subcutaneously administered to the mice for 3 days after the day of operation as a solution or suspension in 0.5% arabic gum dissolved in saline. Unless otherwise specified, fumagillin was given at a dose of 100 mg (10 ml)/kg body weight. Four days later, the mice were given an intra arterial injection of carmine-gelatin solution under Nembutal anesthesia, and cooled on ice for about 2 hours to let the gelatin-containing blood form gels according to the method of Kimura (Kimura, M. et al.; J. Pharmacobiol. Dyn., 9, 442 (1986)). The skin was incised widely over the chambers and inner surface of the skin was exposed. The mouse fascia was observed under a stereoscope (X20; SMZ-10, Nikon). The anti-angiogenic activity of the compounds was evaluated by determining the extent of the tumor-induced vascular formation in mice administered the vehicle or the test compounds.

Tumor-induced vasculature exhibiting both coiling vessels and an increase of the vascular net was defined as positive angiogenesis.

As the results in Table 3 show, fumagillin effectively inhibited the tumor-induced vascular formation.

**Table 3**

| Inhibition of angiogenesis induced by tumor cells by fumagillin | |
|---|---|
| | tumor-induced angiogenesis (positive mice/total mice tested) |
| vehicle (arabic gum-saline) | 5/6 |
| fumagillin treated (100 mg/kg, s.c.) | 0/5 |

### Example 4

### Rat corneal micropocket assay

The rat corneal micropocket assay was carried out essentially by the method of Gimbrone et al. (Gimbrone, M.A. Jr. et al., J. Natl. Cancer Inst. 52, 413(1974)). Corneal vascularization was induced in adult male rats of the Sprague-Dawley strain (Charles River, Japan) by implanting 500 ng of basic FGF (bovine, R & D Systems, Inc.) impregnated in EVA (ethylene-vinyl acetate copolymer) pellets in each cornea (n=5). On the same day, fumagillin (5 µg/pellet) was also impregnated in EVA and implanted into the same cornea between the FGF pellet and the limbus. Control rats received an implantation of the FGF pellet and the EVA pellet without fumagillin. Neovascular formation was observed under a stereoscope (X20, SMZ-10, Nikon). The extent of vascularization was graded from - to +++ (-, without neovascularization; +, positive neovascularization not yet reaching the FGF pellet; ++, positive neovascularization reaching the FGF pellet; +++, positive neovascularization surrounding the FGF pellet).

As shown in Fig. 1, neovascularization stimulated by bFGF was effectively inhibited by fumagillin.

### Example 5

In a similar experiment approximately 10 to 16 chick embryos (age 6 days) were used to assay each concentration of fumagillin substantially in accordance with the method described in Example 2. The fumagillin was applied in a 10µl pellet to the chorioallantoic membrane with or without cyclodextrin. Forty-eight hours later, the percent of embryos with an avascular zone was recorded. The results are shown in Table 4. Unless otherwise indicated, the avascular zones are 1+, e.g., at least 2mm in diameter. An avascular zone of 2+ equals 4mm or greater in diameter, indicating very high activity. For example, 5µg of fumagillin produced 33% avascular zones. In contrast, 5µg of fumagillin plus 25µg of beta-cyclodextrin tetradecasulfate produced 100% avascular zones (the majority of which are 2+). Cyclodextrin (25ug) alone had no detectable effect. Heparin also potentiates fumagillin, but it is not as potent as beta-cyclodextrin in this respect (data not shown).

**Table 4**

| Fumagillin µg/10µl | β-Cyclodextrin tetradecasulfate µg/10 µl | Percent Avascular Zones |
|---|---|---|
| 50 | 0 | 57% |
| 40 | 0 | 72% |
| 30 | 0 | 62% |
| 20 | 0 | 75% |
| 10 | 0 | 75% |
| 5 | 0 | 33% |
| 2.5 | 0 | 40% |
| 1 | 0 | 0% |
| 5 | 25 | 100% (2+) |
| 2.5 | 25 | 57% |
| 1 | 25 | 33% |
| Fumagillin | Heparin | |
| 10 | 50 | 100% |
| β-cyclodextrin alone = 0% avascular zones. Heparin alone = 0% avascular zones. | | |

### Example 6

A pharmaceutical preparation for use as eye-drops was prepared as follows:

| | |
|---|---|
| fumagillin sodium salt | 1 g |
| boric acid | 16 g |
| sodium borate | 7 g |
| p-hydroxy-benzoic acid methylester | 0.25 g |
| p-hydroxy-benzoic acid propionylester | 0.15 g |

Sterile distilled water was added to total 1 liter. After sterilization by filtration, the solution was used as eye-drops.

### Example 7

A pharmaceutical preparation for use as eye-drops was prepared as follows:

| | |
|---|---|
| fumagillin sodium salt | 5 g |
| p-hydroxy-benzoic acid methylester | 0.25 g |
| p-hydroxy-benzoic acid propionylester | 0.15 g |
| dibasic sodium phosphate | 4.g |
| sodium chloride | 8.5 g |

Sterile distilled water was added to total 1 liter. The pH was adjusted to a pH 7.5. After sterilization by filtration, the solution was used as eye-drops.

## Claims (Claims for the following Contracting State(s): AT, BE, CH, DE, FR, GB, IT, LI, LU, NL, SE)

1. Use of fumagillin or a pharmaceutically acceptable salt thereof for preparing a medicament for inhibiting or preventing angiogenesis.

2. Use of fumagillin or a pharmaceutically acceptable salt thereof in conjunction with an agent which potentiates angiogenesis inhibition for preparing a medicament for inhibiting or preventing angiogenesis.

3. Use according to claim 2 wherein the agent which potentiates angiogenesis inhibition is selected from the group of heparin and beta cyclodextrin tetradecasulfate.

4. A pharmaceutical composition for the treatment of angiogenesis in mammals comprising fumagillin or a pharmaceutically acceptable salt thereof and an agent which potentiates angiogenesis inhibition.

5. The pharmaceutical composition of claim 4, wherein the agent which potentiates angiogenesis inhibition is selected from the group of heparin and beta cyclodextrin tetradecasulfate.

## Claims (Claims for the following Contracting State(s): ES, GR)

1. Use of fumagillin or a pharmaceutically acceptable salt thereof for preparing a medicament for inhibiting or preventing angiogenesis.

2. Use of fumagillin or a pharmaceutically acceptable salt thereof in conjunction with an agent which potentiates angiogenesis inhibition for preparing a medicament for inhibiting or preventing angiogenesis.

3. Use according to claim 2 wherein the agent which potentiates angiogenesis inhibition is selected from the group of heparin and beta cyclodextrin tetradecasulfate.

## Patentansprüche (Patentansprüche für folgende(n) Vertragsstaat(en): AT, BE, CH, DE, FR, GB, IT, LI, LU, NL, SE)

1. Verwendung von Fumagillin oder eines pharmazeutisch annehmbaren Salzes davon zur Herstellung eines Medikaments zur Hemmung oder Verhinderung von Angiogenese.

2. Verwendung von Fumagillin oder eines pharmazeutisch annehmbaren Salzes davon in Verbindung mit einem Mittel, das die Angiogenesehemmung potenziert, um ein Medikament zur Hemmung oder Verhinderung von Angiogenese herzustellen.

3. Verwendung nach Anspruch 2, worin das Mittel, das die Angiogenesehemmung potenziert, aus der Gruppe enthaltend Heparin und Betazyklodextrintetradecasulfat ausgewählt ist.

4. Pharmazeutische Zusammensetzung zur Behandlung von Angiogenese bei Säugetieren, die Fumagillin oder ein pharmazeutisch annehmbares Salz davon und ein Mittel umfaßt, das die Angiogenesehemmung potenziert.

5. Pharmazeutische Zusammensetzung nach Anspruch 4, worin das Mittel, das die Angiogenesehemmung potenziert, aus der Gruppe enthaltend Heparin und Betazyklodextrintetradecasulfat ausgewählt ist.

## Patentansprüche (Patentansprüche für folgende(n) Vertragsstaat(en): ES, GR)

1. Verwendung von Fumagillin oder eines pharmazeutisch annehmbaren Salzes davon zur Herstellung eines Medikaments zur Hemmung oder Verhinderung von Angiogenese.

2. Verwendung von Fumagillin oder eines pharmazeutisch annehmbaren Salzes davon in Verbindung mit einem Mittel, das die Angiogenesehemmung potenziert, um ein Medikament zur Hemmung oder Verhinderung von Angiogenese herzustellen.

3. Verwendung nach Anspruch 2, worin das Mittel, das die Angiogenesehemmung potenziert, aus der Gruppe enthaltend Heparin und Betazyklodextrintetradecasulfat ausgewählt ist.

## Revendications (Revendications pour l'(les) Etat(s) contractant(s) suivant(s): AT, BE, CH, DE, FR, GB, IT, LI, LU, NL, SE)

1. Utilisation de la fumagilline ou d'un sel pharmaceutiquement acceptable de cette dernière pour la préparation d'un médicament destiné à inhiber ou à empêcher l'angiogénèse.

2. Utilisation de la fumagilline ou d'un sel pharmaceutiquement acceptable de cette dernière, en combinaison avec un agent qui renforce l'inhibition de l'angiogénèse, pour la préparation d'un médicament destiné à inhiber ou à empêcher l'angiogénèse.

3. Utilisation selon la revendication 2, dans laquelle l'agent qui renforce l'inhibition de l'angiogénèse, est choisi parmi l'héparine et le tétradécasulfate de béta-cyclodextrine.

4. Composition pharmaceutique pour le traitement de l'angiogénèse chez les mammifères, comprenant de la fumagilline ou un sel pharmaceutiquement acceptable de cette dernière, et un agent qui renforce l'inhibition de l'angiogénèse.

5. Composition pharmaceutique selon la revendication 4, dans laquelle l'agent qui renforce l'inhibition de l'angiogénèse, est choisi parmi l'héparine et le tétradécasulfate de béta-cyclodextrine.

## Revendications (Revendications pour l'(les) Etat(s) contractant(s) suivant(s): ES, GR)

1. Utilisation de la fumagilline ou d'un sel pharmaceutiquement acceptable de cette dernière pour la préparation d'un médicament destiné à inhiber ou à empêcher l'angiogénèse.

2. Utilisation de la fumagilline ou d'un sel pharmaceutiquement acceptable de cette dernière, en combinaison avec un agent qui renforce l'inhibition de l'angiogénèse, pour la préparation d'un médicament destiné à inhiber ou à empêcher l'angiogénèse.

3. Utilisation selon la revendication 2, dans laquelle l'agent qui renforce l'inhibition de l'angiogénèse, est choisi parmi l'héparine et le tétradécasulfate de béta-cyclodextrine.
